# EUROPEAN PATENT APPLICATION

(11) **EP 3 009 500 A1**
(43) Date of publication of application: **20.04.2016**
(21) Application number: 15189924.2
(22) Date of filing: 15.10.2015
(51) Int. Cl.: C12M 1/00, C12M 1/34, G01N 15/14

(54) **CULTERE OBERSERVATION APPARATUS AND CULTURE OBSERVATION SYSTEM**

(30) Priority: 17.10.2014 JP 2014212733
(71) Applicant: Olympus Corporation, Tokyo 151-0072 (JP)
(72) Inventor: KIMURA, Hiroyuki, Tokyo, 151-0072 (JP)
(74) Representative: Gunzelmann, Rainer

(57) **Abstract**

An object is to lessen a troublesome checking task during cell culturing. A culture observation apparatus (1) of the present invention includes: a light source unit (3) that radiates illumination light onto the inside of a transparent culture vessel (C) from a side surface of the culture vessel (C) ; an image acquisition unit (4) that images, of the illumination light radiated from the light source unit (3), scattered light emitted from the inside of the culture vessel (C) to acquire images; and a transmission unit (5) that sends the images acquired by the image acquisition unit (4) to the outside. The image acquisition unit (4) includes a sensor array (7) that has a plurality of sensors (8) arranged in at least one line over approximately the entire length in one direction of a bottom surface of the culture vessel (C) and a movement mechanism (9) that relatively moves the sensor array (7) and the culture vessel (C) in a direction intersecting the array direction of the sensors (8) and extending along the bottom surface.

## Description

### {Technical Field}

The present invention relates to a culture observation apparatus and a culture observation system. {Background Art}

In cell culturing, every time cells become confluent, the following process is repeated: a culture vessel is taken out from an incubator, the cells are removed from the culture vessel, and the cells are seeded in a new culture vessel and cultured therein. An observer needs to do this process by checking the cells in the culture vessel in the incubator once or twice a day, which is very troublesome.

Furthermore, a culture reactor in which a transparent conductive film is disposed on a cell contact surface is known (for example, see PTL 1). According to this culture reactor, it is possible to observe the electrical activity of cells being cultured in the incubator.

### {Citation List}

### {Patent Literature}

{PTL 1} Japanese Unexamined Patent Application, Publication No. 2012-80869

### {Summary of Invention}

### {Technical Problem}

From the cells' electrical activity detected by the transparent conductive film of the culture reactor disclosed in PTL 1, it is impossible to observe the number of cells spreading and growing on the bottom surface of the culture vessel and the adhesion area thereof. Therefore, it is impossible to determine whether the cells become confluent.

The present invention has been made in view of the above-described circumstances and provides a culture observation apparatus and a culture observation system capable of lessening a troublesome checking task during cell culturing.

### {Solution to Problem}

According to one aspect, the present invention provides a culture observation apparatus including: a light source unit that radiates illumination light onto an inside of a transparent culture vessel from a side surface of the culture vessel; an image acquisition unit that images, of the illumination light radiated from the light source unit, scattered light emitted from the inside of the culture vessel to acquire images; and a transmission unit that sends the images acquired by the image acquisition unit to the outside, in which the image acquisition unit includes a sensor array that has a plurality of sensors arranged in at least one line over approximately the entire length in one direction of a bottom surface of the culture vessel and a movement mechanism that relatively moves the sensor array and the culture vessel in a direction intersecting the array direction of the sensors and extending along the bottom surface.

According to this aspect, illumination light emitted from the light source unit enters the transparent culture vessel from a side surface of the culture vessel, in which cells are cultured, thereby causing the illumination light to scatter at the cells. This scattered light is emitted to the outside from the bottom surface of the culture vessel, and the image acquisition unit performs imaging to acquire images, thereby making it possible to detect the culture states of the cells in the culture vessel.

Specifically, the sensor array, which has a plurality of sensors arranged in at least one line over approximately the entire length in one direction of the bottom surface of the culture vessel, and the culture vessel are relatively moved, through activation of the movement mechanism, in the direction intersecting the array direction of the sensors and extending along the bottom surface, thereby making it possible to easily acquire images of approximately the entire bottom surface of the culture vessel. Then, the acquired images are sent to the outside by the transmission unit; therefore, for example, when the sent images are received outside the incubator, which accommodates the culture vessel, the culture states of the cells in the culture vessel can be checked without opening the lid of the incubator and taking out the culture vessel. Accordingly, a troublesome checking task during cell culturing can be lessened.

In the above-described aspect, the light source unit may radiate illumination light along an optical axis that falls within a range of ±30° with respect to the horizontal direction.

By doing so, illumination light from the light source unit provides dark-field illumination or oblique illumination, thus making it possible to form shadows on the cells being cultured in the culture vessel. Accordingly, stereoscopic images of the cells are acquired by the image acquisition unit, thus making it possible to observe the culture states of the cells more clearly.

In the above-described aspect, the light source unit may make illumination light enter at a height between the bottom surface of the culture vessel and a solution level of a culture solution reserved in the culture vessel.

By doing so, when illumination light enters the culture vessel, the illumination light does not pass through the solution level of the culture solution or the bottom surface of the culture vessel; therefore, it is possible to make the illumination light propagate through a wider range in the culture vessel.

In the above-described aspect, the sensors may be each provided with a microlens that is disposed facing the bottom surface of the culture vessel and an image acquisition device that is disposed at the opposite side of the microlens from the bottom surface of the culture vessel.

By doing so, an image of cells is formed on the image acquisition device by the microlens, thus making it possible to acquire a clear image. The focal length of the microlens can be set sufficiently short, thus making it possible to reduce the size in the height direction, making the apparatus compact.

In the above-described aspect, the movement mechanism may move the sensor array and the light source unit, and the culture vessel, relatively, in a direction intersecting the array direction of the sensors and extending along the bottom surface.

By doing so, the light source unit, which makes illumination light enter the culture vessel from the side surface of the culture vessel, and the image acquisition unit, which images scattered light that is scattered at cells and emitted to the outside from the bottom surface of the culture vessel, are always maintained in a constant positional relationship; therefore, imaging can always be performed under appropriate illumination. Furthermore, illumination required for imaging performed by the image acquisition unit can be ensured by a small number of light source units.

According to another aspect, the present invention provides a culture observation system including: the above-described culture observation apparatus that is disposed in an incubator; and a receiving unit that is disposed outside the incubator and that receives images sent from the transmission unit of the culture observation apparatus.

By doing so, the culture observation apparatus, which has the culture vessel placed therein, is accommodated in the incubator, images of the bottom surface of the culture vessel acquired periodically or as needed are sent to the outside of the incubator by the transmission unit, and the images are received outside the incubator by the receiving unit, thereby making it possible to observe the culture states of the cells in the incubator, without opening the door of the incubator.

In the above-described aspect, it is possible to further include a determination unit that determines culture states of cells based on the images received by the receiving unit.

By doing so, the determination unit determines the culture states of the cells based on the images, thus making it possible to easily determine whether the cells are confluent.

### {Advantageous Effects of Invention}

According to the present invention, an advantage is afforded in that a troublesome checking task during cell culturing can be lessened.

### {Brief Description of Drawings}

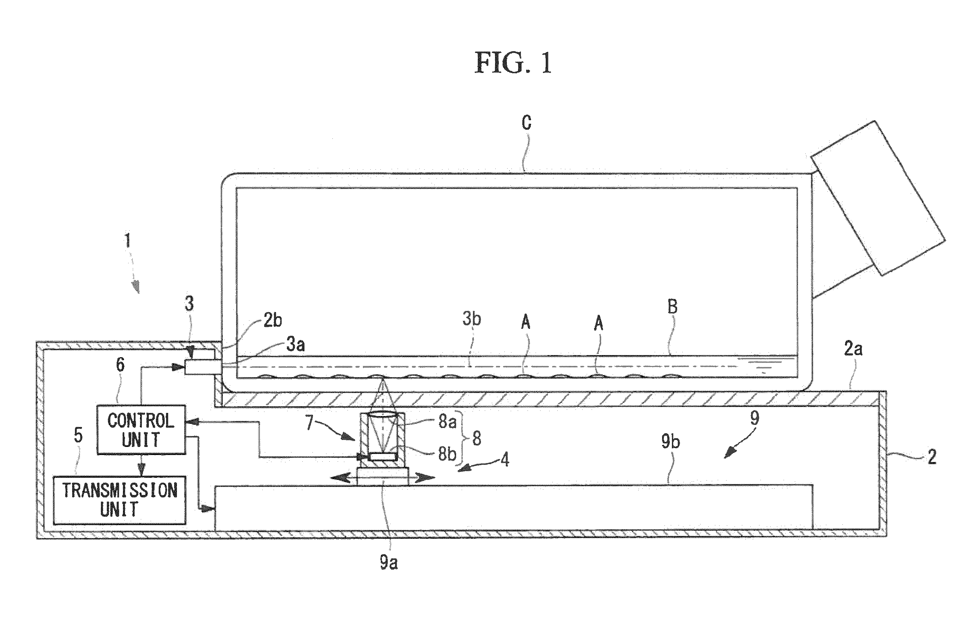

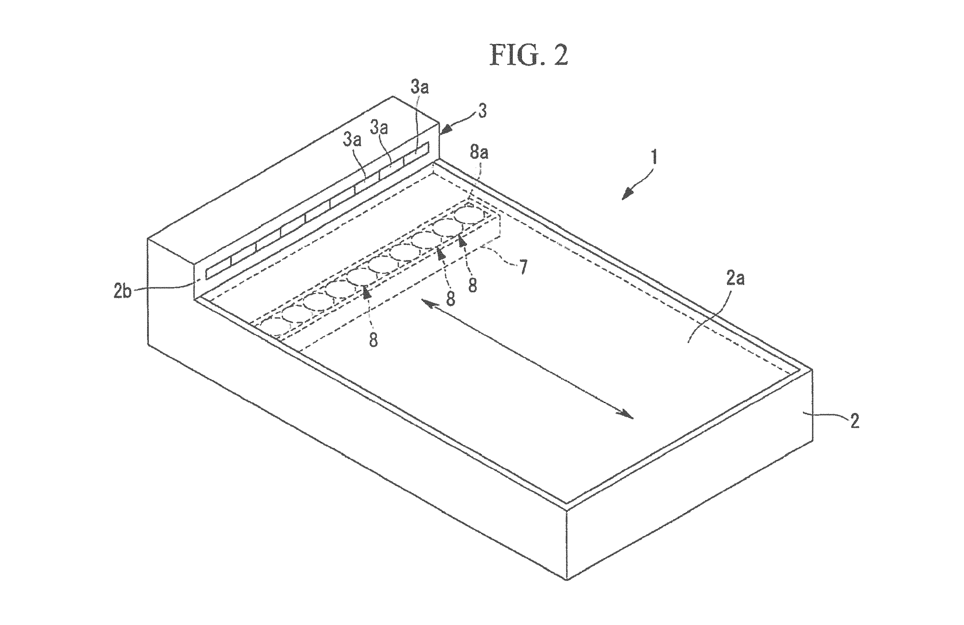

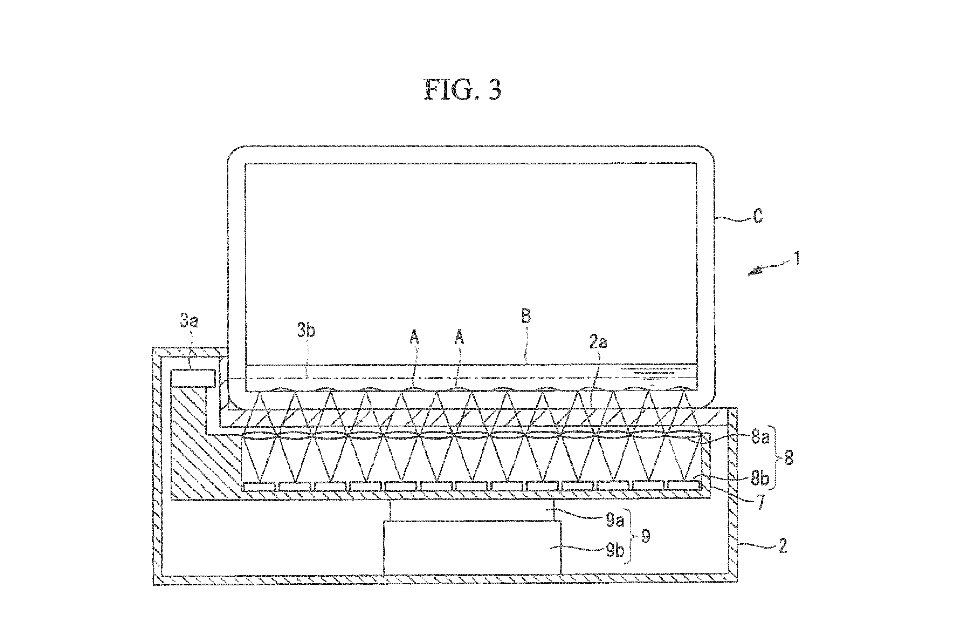

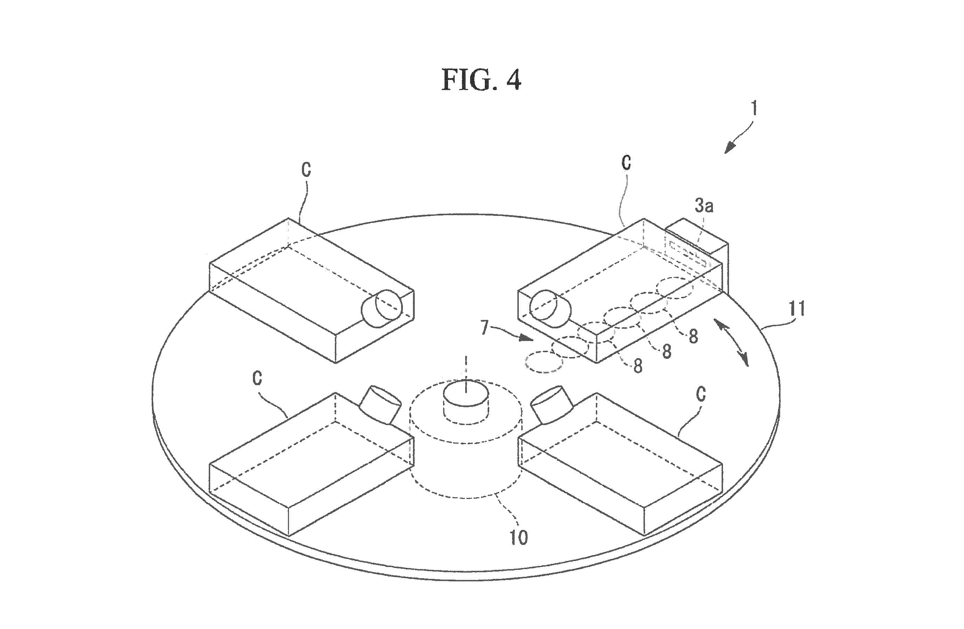

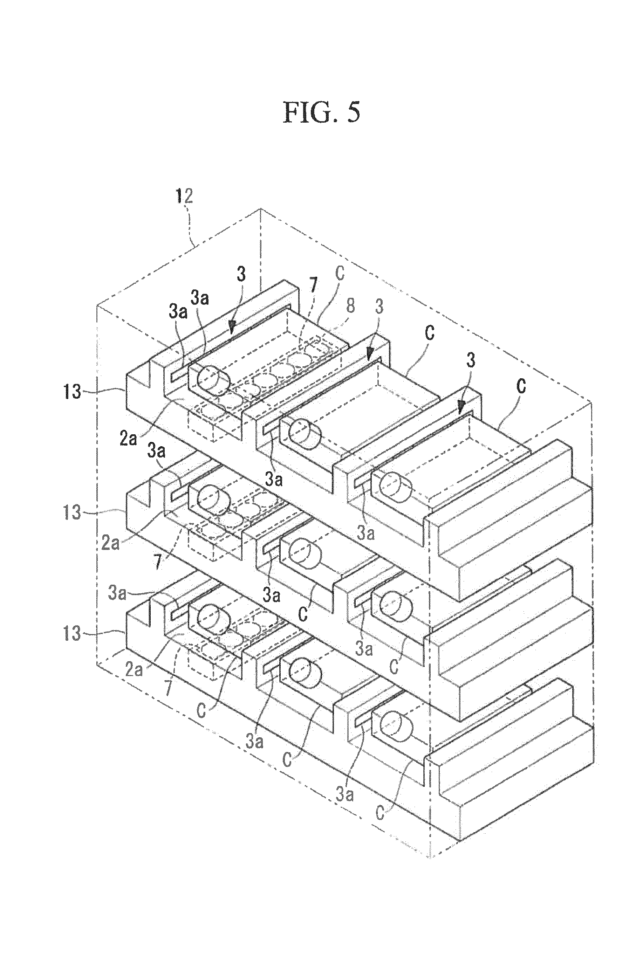

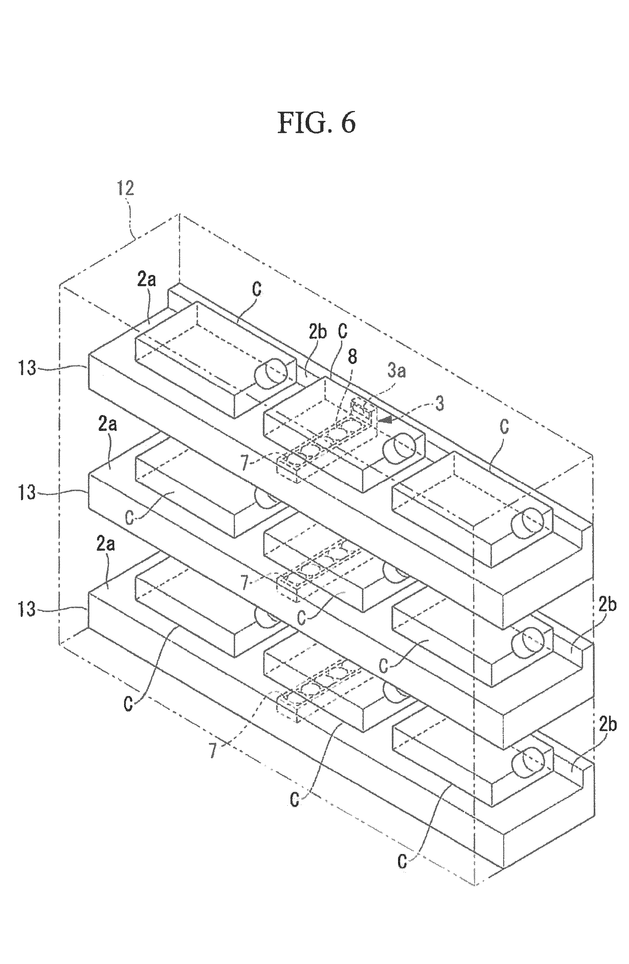

{Fig. 1} Fig. 1 is a longitudinal sectional view showing a culture observation apparatus according to one embodiment of the present invention.
{Fig. 2} Fig. 2 is a perspective view showing the culture observation apparatus shown in Fig. 1.
{Fig. 3} Fig. 3 is a transverse sectional view showing a modification of the culture observation apparatus shown in Fig. 1.
{Fig. 4} Fig. 4 is a perspective view showing another modification of the culture observation apparatus shown in Fig. 1.
{Fig. 5} Fig. 5 is a perspective view showing still another modification of the culture observation apparatus shown in Fig. 1.
{Fig. 6} Fig. 6 is a perspective view showing still another modification of the culture observation apparatus shown in Fig. 1.

### {Description of Embodiment}

A culture observation apparatus 1 according to one embodiment of the present invention will be described below with reference to the drawings.

As shown in Fig. 1, the culture observation apparatus 1 of this embodiment includes a base 2 on which is placed a culture vessel C that accommodates, together with culture solution B, cells A to be cultured, and a light source unit 3, an image acquisition unit 4, a transmission unit 5, and a control unit 6 that are provided in the base 2.

The culture vessel C is, for example, a flask for cell culturing and is made of an optically-transparent material.

As shown in Figs. 1 and 2, the base 2 has a placement surface 2a with which a lower surface of the culture vessel C is brought into close contact and that is made of an optically-transparent material and an abutment surface 2b that rises vertically from the placement surface 2a and with which one side surface of the culture vessel C, which is placed on the placement surface 2a, is brought into close contact. Since the inside of an incubator is humid, the base 2 has a waterproof structure.

The light source unit 3 is disposed on the abutment surface 2b and has a plurality of LED light sources 3a arranged above the placement surface 2a with a predetermined space therebetween and in a direction parallel to the placement surface 2a. The predetermined space from the placement surface 2a is set so as to be equal to or slightly larger than the distance from the lower surface of the culture vessel C to be placed thereon to a bottom surface of the inside of the culture vessel C and so as to be smaller than the distance from the lower surface of the culture vessel C to a solution level of the culture solution B, which is expected to be reserved in the culture vessel C.

Furthermore, an optical axis 3b of illumination light emitted from each of the LED light sources 3a is set so as to be approximately parallel to the placement surface 2a.

The image acquisition unit 4 includes a sensor array 7 that is disposed in the base 2 below the placement surface 2a and a movement mechanism 9 that moves the sensor array 7. The sensor array 7 has a plurality of sensors 8 arranged in a line. The sensors 8 each include a light collecting lens 8a that is disposed facing a bottom surface of the placement surface 2a and an image acquisition device 8b that images light collected by the light collecting lens 8a to acquire an image.

The sensor array 7 is disposed over approximately the same length as that of a shorter side of the bottom surface of the culture vessel C so as to be parallel to the shorter side. Furthermore, imaging ranges of adjacent image acquisition devices 8b are arranged adjacent to each other, and thus, when detection is performed by all of the sensors 8 with the sensor array 7 fixed in place, a row of continuous images can be acquired along the array direction of the sensors 8 over approximately the entire length of the shorter side of the bottom surface of the culture vessel C.

The movement mechanism 9 includes a slider 9a that fixes the sensor array 7 and a linear motion mechanism 9b that linearly moves the slider 9a in a direction that is perpendicular to the array direction of the sensors 8 in the sensor array 7 and that extends along the placement surface 2a. The linear motion mechanism 9b may be constituted, for example, of a motor and ball screws, which are not shown.

The transmission unit 5 wirelessly sends images acquired by the image acquisition devices 8b to the outside.

Furthermore, the control unit 6 has, for example, a timer, which is not shown, and periodically activates the light source unit 3, the image acquisition unit 4, and the transmission unit 5.

The operation of the thus-configured culture observation apparatus 1 of this embodiment will be described below.

To observe the culture states of the cells A by using the culture observation apparatus 1 of this embodiment, the culture vessel C, which accommodates the cells A to be cultured and the culture solution B, is placed on the base 2 such that the lower surface of the culture vessel C is brought into close contact with the placement surface 2a of the base 2 and such that one side surface of the culture vessel C is abutted against the abutment surface 2b of the base 2.

In this state, the culture observation apparatus 1, which has the culture vessel C placed therein, is accommodated in an incubator (not shown) and is disposed such that the placement surface 2a becomes horizontal, thereby starting culturing of the cells A in the culture vessel C under an environment in which the temperature and the humidity in the incubator are managed. Furthermore, at this time, the timer in the control unit 6 is activated to start timekeeping.

After culturing is started, based on a schedule set in advance according to the timekeeping result of the timer, the control unit 6 activates the light source unit 3 to turn on the LED light sources 3a and also causes the image acquisition devices 8b to perform imaging.

The LED light sources 3a are provided on the abutment surface 2b, against which the side surface of the culture vessel C is pushed, and make illumination light enter the culture vessel C from a side surface of the culture vessel C in the directions of the optical axes 3b along the bottom surface of the culture vessel C. Thus, as in oblique illumination or dark-field illumination, the cells A adhering to and growing on the bottom surface of the culture vessel C are illuminated from a lateral side, thus forming shadows of the cells A.

Part of the scattered light scattered at the cells A is transmitted through the bottom surface of the culture vessel C and the placement surface 2a of the base 2 and is collected by the light collecting lenses 8a in the base 2, thus being imaged by the image acquisition devices 8b. In this case, with the culture observation apparatus 1 of this embodiment, because the sensor array 7 is constituted of the plurality of sensors 8 arranged in a line, a row of sharp-focused images are acquired at each point in time. Then, the control unit 6 activates the movement mechanism 9 to move the sensor array 7 in the direction perpendicular to the array direction of the sensors 8 and, meanwhile, causes the image acquisition devices 8b to perform imaging, thereby making it possible to acquire a plurality of rows of images. The interval between imaging actions performed by the image acquisition devices 8b is set sufficiently short, thereby making it possible to acquire images over almost all regions of the bottom surface of the culture vessel C, which is placed on the placement surface 2a.

Every time images are acquired over almost all of the regions of the bottom surface of the culture vessel C, the control unit 6 turns off the LED light sources 3a. In this way, the light source unit 3 etc. is activated intermittently, thereby making it possible to suppress a temperature increase of the apparatus to reduce the influence of heat on the cells.

Then, the images acquired by the image acquisition devices 8b are sent from the control unit 6 to the transmission unit 5 and are sent to the outside by the transmission unit 5. Therefore, when the images sent from the transmission unit 5 are received outside the incubator and displayed on a monitor, the culture states of the cells A in the culture vessel C can be checked outside the incubator without taking out the culture vessel C from the incubator for observation and without even opening the door of the incubator. Specifically, there is the advantage that a troublesome checking task during cell culturing can be significantly lessened. Furthermore, because the culture vessel C does not need to be taken out from the incubator, it is possible to eliminate a change in environment (change in temperature, pH, etc.) for the cells being cultured.

In particular, according to this embodiment, because the light source unit 3 makes illumination light enter the culture vessel C in the direction parallel to the bottom surface thereof, to which the cells A adhere, it is possible to form shadows on the cells A being cultured in the culture vessel C. Accordingly, the image acquisition unit 4 acquires stereoscopic images of the cells A, thus making it possible to more clearly observe the culture states of the cells A.

Furthermore, because the light source unit 3 makes illumination light enter between the bottom surface of the culture vessel C and the solution level of the culture solution B, the illumination light does not pass through the solution level of the culture solution B or the bottom surface of the culture vessel C, thus making it possible to suppress scattering of the illumination light to make the illumination light propagate farther in the culture solution B, which allows illumination over a wide range.

Furthermore, because the image acquisition unit 4 images, among the scattered light scattered at the cells A being cultured, scattered light that has been transmitted through the bottom surface of the culture vessel C, when the cells A are cultured under a high-temperature and high-humidity environment, it is possible to acquire clear images without being affected by water droplets formed on an upper surface of the culture vessel C through dew condensation.

Furthermore, in this embodiment, because the LED light sources 3a are used as the light source unit 3, there is the advantage that heat generation is suppressed, thus making it possible to reduce the influence on cells and also to suppress power consumption.

Note that, in this embodiment, although illumination light from the light source unit 3 enters the culture vessel C in the horizontal direction along the optical axes 3b parallel to the bottom surface, the direction is not limited thereto, and the illumination light may enter at an angle of ±30° or less with respect to the horizontal direction. Even when such an angle is adopted, it is possible to form, on the cells A, shadows similar to those formed by dark-field illumination or oblique illumination and to acquire stereoscopic images.

Furthermore, in this embodiment, although the control unit 6 has the timer and periodically activates the light source unit 3 etc., instead of this, a receiving unit (not shown) may be connected to the control unit 6 to receive a command signal from outside the incubator, and the control unit 6 may drive the light source unit 3 etc. in response to the command signal. By doing so, an operator can turn on/off the light source unit 3 and perform imaging at a desired timing by remote control.

Transmission and reception of image signals and command signals can be performed via radio or wires.

Furthermore, in this embodiment, the side surface of the culture vessel C at the opposite side from an opening provided on the culture vessel C for taking the culture solution B and the cells A in and out (the shorter side surface of the bottom surface) is abutted against the abutment surface 2b of the base 2, and the light source unit 3 is provided on the abutment surface 2b; however, the light source unit 3 may be disposed along a longer side surface of the bottom surface.

Furthermore, in this embodiment, because the sensor array 7 is disposed in the direction along the shorter side of the bottom surface of the culture vessel C and is moved in the direction along the longer side of the bottom surface, the number of sensors 8 required for the sensor array 7 is reduced, thus allowing cost reduction. Instead of this, the sensor array 7 may be disposed in the direction along the longer side of the bottom surface of the culture vessel C and moved in the direction along the shorter side of the bottom surface. By doing so, although the number of sensors 8 required for the sensor array 7 is increased, there is the advantage that the movement distance of the movement mechanism 9 is shortened, thus making it possible to acquire images over almost all regions of the bottom surface in a shorter time.

Furthermore, in this embodiment, a description has been given of a case in which adjacent sensors 8 can acquire images with no spaces therebetween; however, imaging ranges in which images can be acquired by adjacent sensors 8 may be arranged with spaces therebetween. In that case, the movement mechanism 9 may adopt a configuration in which the sensor array 7 can also be moved in the array direction of the sensor array 7 and may move the sensor array 7 so as to fill the spaces between the imaging ranges, thus performing imaging by a raster scanning method. Accordingly, there is the advantage that the imaging magnification can be increased by limiting the imaging ranges of the sensors 8.

Furthermore, in this embodiment, although the light source unit 3 is fixed, instead of this, as shown in Fig. 3, the light source unit 3 may be fixed to the sensor array 7 and moved by the movement mechanism 9 together with the sensor array 7. In the example shown in Fig. 3, the light source unit 3 is disposed so as to be able to be moved in a base 2 that is sealed by an optically-transparent abutment surface 2b formed integrally with the placement surface 2a and makes illumination light enter the culture vessel C through the abutment surface 2b. The light source unit 3 may make illumination light enter the culture vessel C from one side surface of the culture vessel C, as shown in Fig. 3, or from both side surfaces thereof.

Furthermore, instead of the sensor array 7, in which a plurality of sensors 8 each consisting of the light collecting lens 8a and the image acquisition device 8b are arranged, it is possible to adopt a line sensor in which a number of pixels are arranged in a line.

Furthermore, instead of moving the sensor array 7 in the base 2, it is also possible to fix the sensor array 7 and the light source unit 3 and to move the culture vessel C. For example, as shown in Fig. 4, it is possible that, above the sensor array 7, one or more culture vessels C are arranged on a turntable 11 that is provided so as to be able to be horizontally rotated by a motor 10, and the turntable 11 is rotated, thereby moving the culture vessels C with respect to the sensor array 7 and acquiring images of approximately the entire bottom surfaces of the culture vessels C.

Instead of the turntable 11, a linearly-moving table may be adopted.

Furthermore, in this embodiment, although the culture observation apparatus 1, having a configuration in which the culture vessel C is placed on the placement surface 2a, and the culture observation apparatus 1 is accommodated in an incubator 12 together with the culture vessel C, is shown as an example, instead of this, as shown in Figs. 5 and 6, it is possible to dispose, in the form of the culture observation apparatus 1 having the above-described configuration, shelf boards 13 that are provided in the incubator 12. In the figures, only inner volume portions of the incubator 12 are indicated by solid lines to make the inner configuration clear.

For example, as shown in Fig. 5, each shelf board 13 on which a plurality of (in Fig. 5, three) culture vessels C can be placed may have the light source units 3, which are fixed above the placement surface 2a, and the sensor array 7, which can be linearly moved below the placement surface 2a, as in Fig. 2. In Fig. 5, the shelf boards 13 are provided at three stages. The light source units 3 are fixed above the placement surface 2a with spaces slightly larger than the shorter sides of the bottom surfaces of the culture vessels C and make illumination light enter the culture vessels C inserted between the light source units 3, from the side surfaces of the culture vessels C.

The sensor array 7 accommodated in each of the shelf boards 13 is linearly moved so as to sequentially scan the three culture vessels C on the shelf board 13, thus acquiring images of the bottom surfaces of the culture vessels C. At this time, each of the light source units 3 may be controlled so as to radiate illumination light onto the corresponding culture vessel C only when the sensor array 7 is disposed below the culture vessel C. Furthermore, activation of the light source unit 3 and/or activation of the sensor array 7 may be stopped at a portion where the culture vessel C is not placed.

Furthermore, for example, as shown in Fig. 6, each shelf board 13 on which a plurality of (in Fig. 6, three) culture vessels C can be placed may accommodate the light source unit 3 that radiates illumination light from the inner side of the abutment surface 2b, against which the longer-side surfaces of the bottom surfaces of the culture vessels C are pushed, and the sensor array 7, which acquires images below the placement surface 2a, in a manner allowing them to be linearly moved, as in Fig. 3. By disposing a plurality of culture vessels C such that the side surfaces thereof are pushed against the abutment surface 2b, which is located on the far side of the incubator 12, it is possible to sequentially perform radiation of illumination light and imaging of the bottom surfaces according to the movement of the sensor array 7 and the light source unit 3.

The number of shelf boards 13 and the number of culture vessels C that can be placed on one shelf board 13 may be set as desired.

The light source unit 3 may make illumination light enter the culture vessel C from one side surface of the culture vessel C, as shown in Fig. 6, or from both side surfaces thereof.

Furthermore, for example, in the modifications shown in Figs. 5 and 6 in which the shelf boards 13 in the incubator 12 are provided with the linearly-movable sensor arrays 7, the shelf boards 13 may be inclined by using movable supporting columns (not shown) or the like, thereby gravitationally moving the sensor arrays 7.

Furthermore, in this embodiment, although a description has been given of the culture observation apparatus 1, which is accommodated in the incubator 12 together with the culture vessel C, it is desirable that the culture observation apparatus 1 be applied to a culture observation system provided with a receiving unit that is disposed outside the incubator 12 and that receives images sent from the transmission unit 5. Furthermore, it is possible to provide, outside the incubator 12, a monitor that displays the images received by the receiving unit, an image processing unit that processes the images received by the receiving unit, or a determination, unit that determines, based on the processed images, the culture states of cells, for example, the number of cells, the adhesion area, or whether the cells become confluent. This culture observation system has the advantage that the culture states can be easily determined without opening and closing the incubator 12.

Furthermore, in this embodiment, the imaging range and the imaging magnification can be desirably set for each of the sensors 8 which constitute the sensor array 7.

### {Reference Signs List}

- B: culture solution
- C: culture vessel
- 1: culture observation apparatus
- 3: light source unit
- 4: image acquisition unit
- 5: transmission unit
- 7: sensor array
- 8: sensor
- 8a: light collecting lens
- 8b: image acquisition device
- 9: movement mechanism
- 10: motor (movement mechanism)
- 11: turntable (movement mechanism)
- 12: incubator

## Claims

1. A culture observation apparatus comprising:
a light source unit that radiates illumination light onto an inside of a transparent culture vessel from a side surface of the culture vessel;
an image acquisition unit that images, of the illumination light radiated from the light source unit, scattered light emitted from the inside of the culture vessel to acquire images; and
a transmission unit that sends the images acquired by the image acquisition unit to the outside,
wherein the image acquisition unit includes a sensor array that has a plurality of sensors arranged in at least one line over approximately the entire length in one direction of a bottom surface of the culture vessel and a movement mechanism that relatively moves the sensor array and the culture vessel in a direction intersecting the array direction of the sensors and extending along the bottom surface.

2. A culture observation apparatus according to claim 1, wherein the light source unit radiates illumination light along an optical axis that falls within a range of ±30° with respect to the horizontal direction.

3. A culture observation apparatus according to claim 1 or 2, wherein the light source unit makes illumination light enter at a height between the bottom surface of the culture vessel and a solution level of a culture solution reserved in the culture vessel.

4. A culture observation apparatus according to one of claims 1 to 3, wherein the sensors are each provided with a microlens that is disposed facing the bottom surface of the culture vessel and an image acquisition device that is disposed at the opposite side of the microlens from the bottom surface of the culture vessel.

5. A culture observation apparatus according to one of claims 1 to 4, wherein the movement mechanism moves the sensor array and the light source unit, and the culture vessel, relatively, in a direction intersecting the array direction of the sensors and extending along the bottom surface.

6. A culture observation system comprising:
a culture observation apparatus according to one of claims 1 to 5, which is disposed in an incubator; and
a receiving unit that is disposed outside the incubator and that receives images sent from the transmission unit of the culture observation apparatus.

7. A culture observation system according to claim 6, further comprising a determination unit that determines culture states of cells based on the images received by the receiving unit.
